# EUROPEAN PATENT SPECIFICATION

(11) **EP 2 200 514 B1**
(45) Date of publication and mention of the grant of the patent: **04.01.2012**
(21) Application number: 07854394.9
(22) Date of filing: 24.10.2007
(51) Int. Cl.: A61B 6/00, A61B 6/04

(54) **METHOD AND APPARATUS FOR VISUALIZING THE CERVICAL VERTEBRAE IN A SINGLE IMAGE**
VERFAHREN UND GERÄT ZUR VISUALISIERUNG DER HALSWIRBEL IN EINEM EINZELNEN BILD
PROCEDE ET APPAREIL DE VISUALISATION DES VERTEBRES CERVICALES EN UNE SEULE IMAGE

(43) Date of publication of application: 30.06.2010
(73) Proprietor: Citow Cervical Visualizer CO., Libertyville, IL 60048 (US)
(72) Inventor: PICKETT, Jill, C., Hobart IN 46342 (US); CITOW, Jonathan, S., Glencoe IL 60025 (US)
(74) Representative: Pitchford, James Edward
(86) International application number: PCT/US2007/082416
(87) International publication number: WO 2009/054850

(56) References cited:
- EP-A2- 1 264 586
- JP-A- 1 265 944
- JP-U- 54 002 379
- JP-U- 58 160 007
- US-A- 3 700 894
- US-A- 5 342 290
- US-A1- 2007 144 530

## Description

### BACKGROUND

The human spine or backbone is known medically as the vertebral column. Its role is to support the whole body, be capable of bending and twisting in all directions, and at the same time protect the vital structures such as nerves that run through it.

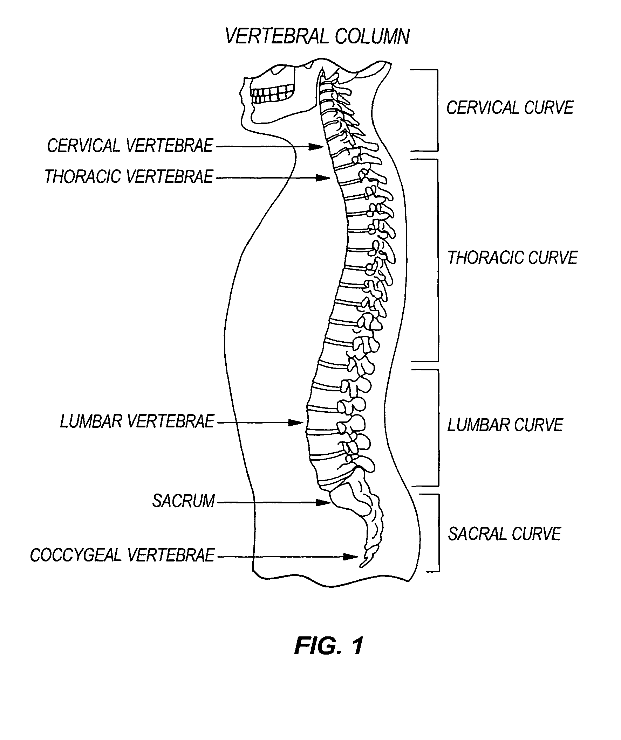

As illustrated in FIG. 1, the human spine consists of a column of bony blocks known as vertebrae, which sit one on top of another, joined together by tough ligaments to form the vertebral column. The spine is divided into several sections known as the cervical vertebrae, the thoracic vertebrae, the lumbar vertebrae, the sacrum, and the coccygeal vertebrae.

The cervical vertebrae include seven vertebra and eight pairs of cervical nerves. The cervical vertebrae are those vertebrae in the neck, immediately behind the skull. The individual cervical vertebrae are identified as C1 through C7. The cervical nerves are also identified as C1 through C8. The thoracic vertebrae include twelve vertebra, identified as T1 through T12, in the upper and middle back. The lumbar vertebrae include five vertebra, identified as L1 through L5, in the lower part of the spine. The fifth lumbar vertebra sits on the sacrum, which in turn is connected to the coccyx, i.e., the tail bone. The sacrum consists of several vertebrae that have joined together. The sacrum is joined at its edges to the pelvis; the ring of bone that carries the trunk and which in turn is supported by the hips.

During anterior cervical disectomy and fusion procedures, X-rays may be necessary. The radiographic area in such a surgical procedure is constrained by anatomical obstruction by the subject's shoulders or the subject's jaw. It is rare that all seven cervical vertebrae are visible in a single X-ray because the jaw of the subject may obstruct the view of C1 and C2, while the shoulders of the subject may obscure C7 and the first thoracic vertebra T1.
Background art is provided in US 2007/144530 A1, which discloses a shoulder press device according to the pre-characterising portion of Claim 1 of the appended claims.

### SUMMARY

This invention relates generally to the field of devices and systems for neurophysiological monitoring, testing, and assessment in both clinical and intraoperative settings.
The present invention is a shoulder press device as defined in Claim 1 of the appended claims.

The present invention provides a device that optimizes intraoperative radiography by removing obstructions in the radiographic area of the cervical vertebrae. The device holds the subject's shoulders in a position so that all of the cervical vertebrae are visible in an X-ray.

The device is a shoulder press for use during the acquisition of X-rays of the cervical or cervical thoracic junction regions of the patient. The device optimizes the radiographic area of the patient during acquisition of X-rays. The device supports the patient's shoulders and adjusts the position of the shoulders such that they do not obstruct the X-ray images. The device is suitable for use in the clinical and ER settings as well as for intraoperative use such as anterior cervical discectomy or fusion procedures.

The invention provides a position device for altering a position of a portion of a patient. The position device comprises an telescoping elongated rod having a first end and a second end, a first handle, a second handle, a first arm, and a second arm. The first handle is coupled to the first end of the elongated rod and includes an elongated portion, an enlarged portion extending from the elongated portion, and an extension extending from the enlarged portion. The extension has a curvature following a downward path toward the second end of the elongated rod. The second handle is coupled to the second end of the elongated rod and includes an elongated portion, an enlarged portion extending from the elongated portion, and an extension extending from the enlarged portion. The extension has a curvature following a downward path toward the first end of the elongated rod. The first arm includes a first end coupled to the elongated rod and a second end and is oriented substantially perpendicular with respect to the elongated rod. The first arm also includes a base having a central portion, a first extension extending from the central portion, and a second extension extending from the central portion, the first extension defining a radius of curvature of a first length, and the second extension defining a radius of curvature of a second length different than the first length. The second arm is spaced from the first arm and includes a first end coupled to the elongated rod and a second end and is oriented substantially perpendicular with respect to the elongated rod. The second arm also includes a base having a central portion, a first extension extending from the central portion, and a second extension extending from the central portion, the first extension defining a radius of curvature of a first length, and the second extension defining a radius of curvature of a second length .

Other aspects of the invention will become apparent by consideration of the detailed description and accompanying drawings.

### BRIEF DESCRIPTION OF THE DRAWINGS

FIG. 1 is a side view of a human spinal spine.

FIG. 2 is top view of a position device according to an embodiment of the present invention.

FIG. 3 is a rear view of the position device illustrated in FIG. 2.

FIG. 4 is a side view of an arm of the position device illustrated in FIG. 2.

FIG. 5 is an enlarged side view of the arm illustrated in FIG. 4.

FIG. 6 is a rear view of the arm illustrated in FIG. 4

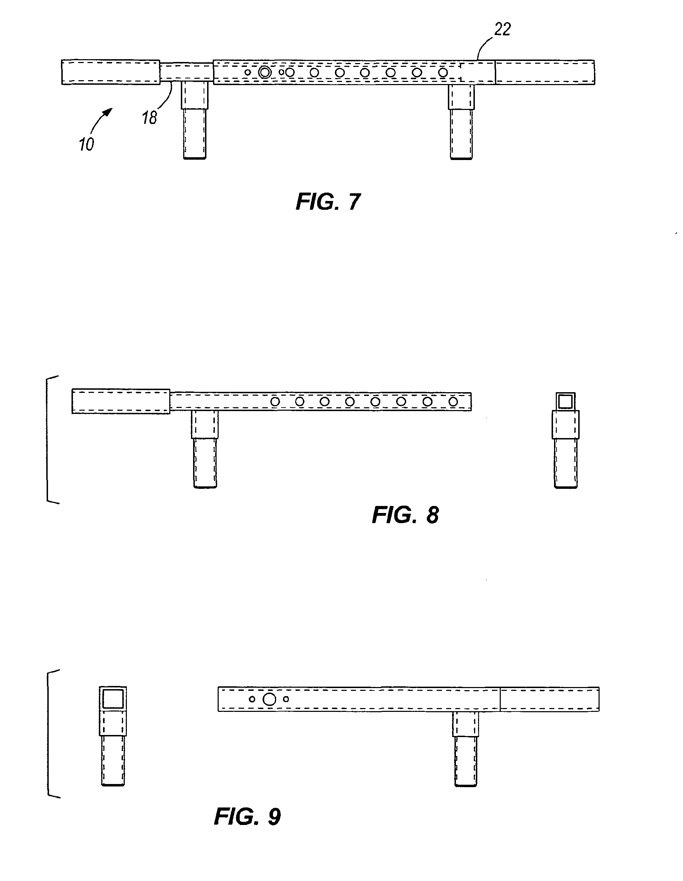

FIG. 7 is a side view of the position device illustrated in FIG. 2

FIG. 8 is a partial side view of the position device illustrated in FIG. 2.

FIG. 9 is a partial side view of the position device illustrated in FIG. 2.

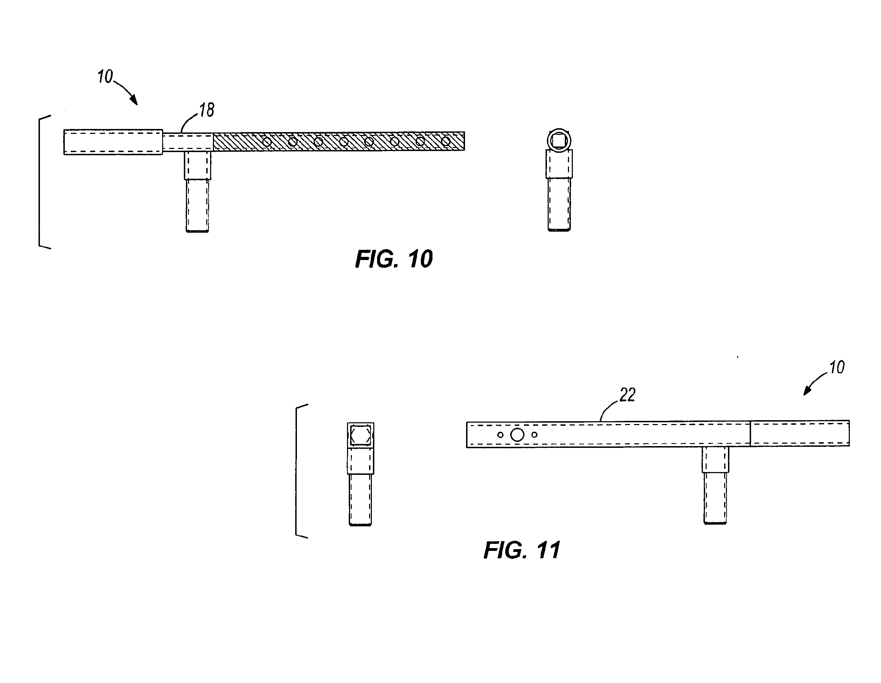

FIG. 10 is a partial side view of the position device illustrated in FIG. 2.

FIG. 11 is a partial side view of the position device illustrated in FIG. 2.

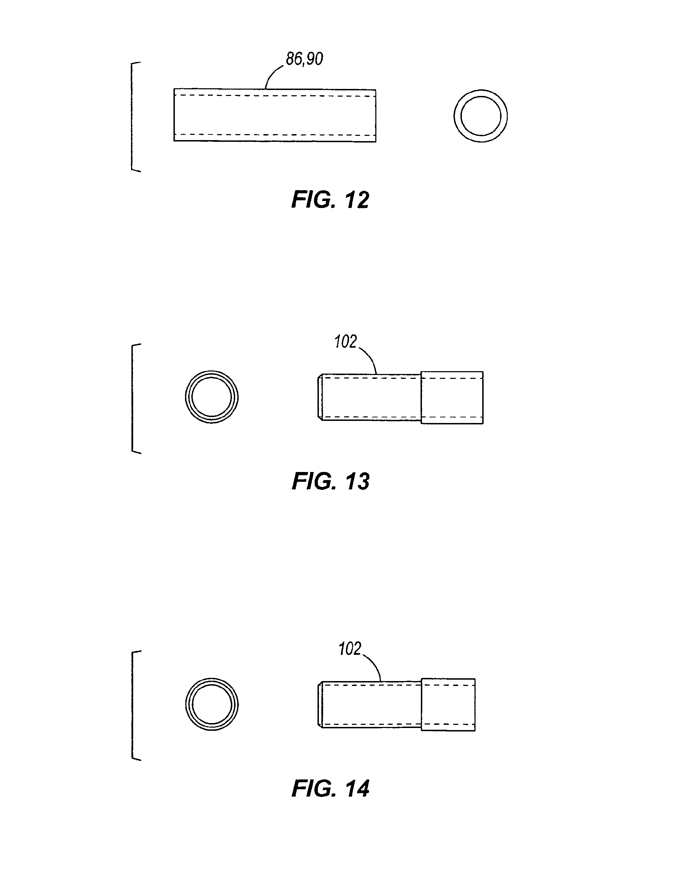

FIG. 12 is a dimensional view of a portion of the position device illustrated in FIG. 2.

FIG. 13 is a dimensional view of a portion of the position device illustrated in FIG. 2.

FIG. 14 is a partial side view of the position device illustrated in FIG. 2.

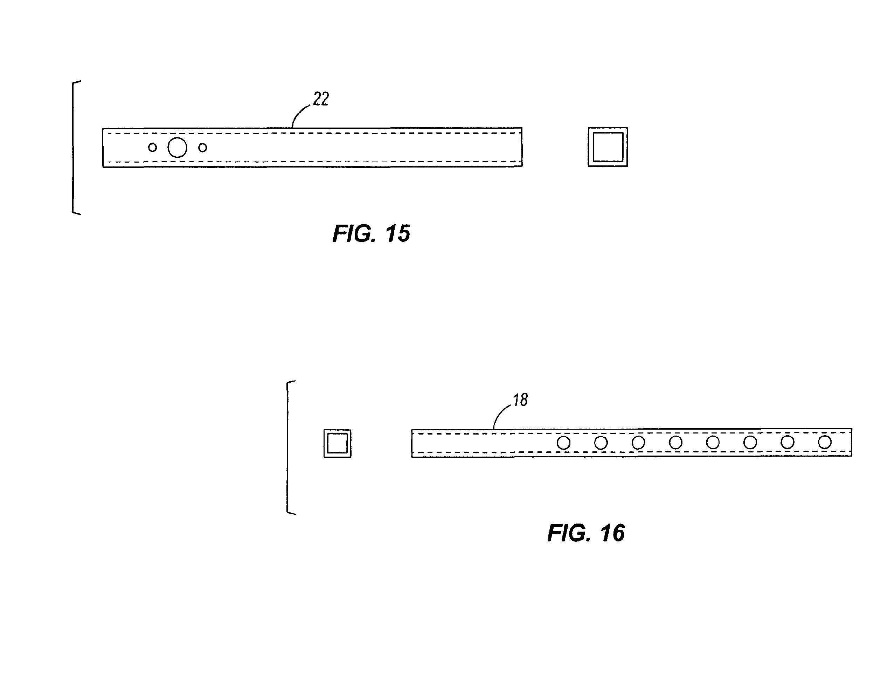

FIG. 15 is a partial side view of the position device illustrated in FIG. 2.

FIG. 16 is a partial side view of the position device illustrated in FIG. 2.

### DETAILED DESCRIPTION

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

Although directional references, such as upper, lower, downward, upward, rearward, bottom, front, rear, etc., may be made herein in describing the drawings, these references are made relative to the drawings (as normally viewed) for convenience. These directions are not intended to be taken literally or limit the present invention in any form. In addition, terms such as "first," "second," and "third" are used herein for purposes of description and are not intended to indicate or imply relative importance or significance.

In intraoperative and trauma situations related to the neck area of the spine, a physician needs to review anterior and lateral X-ray views of the cervical vertebrae. However, the cervical vertebrae are often obscured by the anatomical position of the patient's shoulders and/or jaw. It is rare that all seven cervical vertebra are visible in a single X-ray of the cervical vertebrae and/or cervical-thoracic junction. Typically, the patient's jaw obstructs the cervical vertebrae C1 and C2, and the patient's shoulders obstruct the cervical vertebra C7 and/or the thoracic vertebra T1.

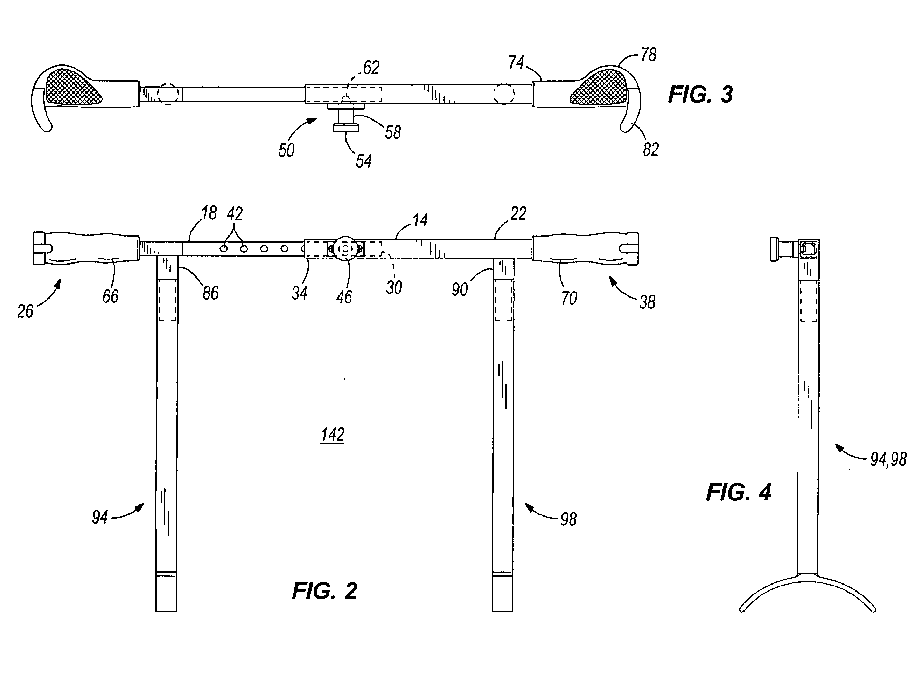

FIG. 2 illustrates a position device 10 according to an embodiment of the present invention. The position device 10 is adapted to push the patient's shoulders caudally to allow a more inclusive view of the cervical vertebrae in anterior and lateral cervical spine films. The position device 10 or at least a portion of the device 10 is comprised of radiolucent carbon fiber material. Other suitable radiolucent materials also can be utilized in the device 10. The radiolucent material prevents the device 10 or portions of the device 10 from appearing in the X-ray film, i.e., the device 10 does not obstruct the radiographic field of view of the image. The position device 10 can provide correct level confirmation following interbody device placement, proper plate placement, and for post procedure confirmation.

The position device 10 includes an elongated rod 14 having a first portion 18 and a second portion 22. The first portion 18 includes a first end 26 and a second end 30 and has a first diameter. The second portion 22 includes a first end 34 and a second end 38 and has a second diameter. The first diameter and the second diameter are different such that the second end 30 of the first portion 18 slides within the first end 34 of the second portion 22. In other words, the elongated rod 14 telescopes to adjust to different lengths. The first portion 18 or the second portion 22 can include bearings to assist the telescoping action. In another construction, the interiors of the first portion 18 and the second portion 22 can include a powder coat to assist the telescoping action. In some constructions, the exteriors of the first portion 18 and the second portion 22 can include a coating, such as chrome or other suitable specialty coating. The coating can assist in the cleaning and/or sterilization process of the device 10.

The first portion 18 includes a plurality of apertures 42 positioned along its length. The second portion 22 includes an aperture 46 positioned near the first end 34. The first portion 18 and the second portion 22 of the elongated rod 14 have a substantially square cross-section, but other shapes for the cross-section are also possible.

The position device 10 also includes a knob 50 coupled to the second portion 22 of the elongated rod 14. The knob 50 includes a base 54, a plunger 58, and a protrusion 62 extending from the plunger 58. The plunger 58 can include a spring 62 or other elastic component. The protrusion 62 is sized to be received with the aperture 46 of the second portion 22 and the apertures 42 of the first portion 18. The knob 50 is manipulated to adjust the length of the elongated rod 14. More specifically, the knob 50 is manipulated to pull the plunger 58 to remove the protrusion 62 from the apertures 42, 46 such that the first portion 18 can telescope within the second portion 22. The knob 50 is then released to position the protrusion 62 into the desired apertures 42, 46 (i.e., the apertures 46 is aligned with one of the apertures 42).

The position device 10 also includes a first handle 66 mounted to the first end 26 of the first portion 18 of the elongated rod 14 and a second handle 70 mounted to the second end 38 of the second portion 22 of the elongated rod 14. The first handle 66 and the second handle 70 include ergonomic grips with lateral stability for proper placement of the device 10. The first handle 66 and the second handle 70 include an elongated portion 74 and an expanded portion 78 extending from and integral to the elongated portion 74. The first handle 66 and the second handle 70 also include an extension 82 extending from and integral to the expanded portion 78. The extension 82 narrows in cross-section as it extends further from the expanded portion 78. The extension 82 curves downward and toward the opposite end of the elongated rod 14. The first handle 66 and the second handle 70 can comprise rubber, silicone, or any other suitable material and/or combination of materials.

The position device 10 includes a first coupling 86 connected to and extending from the first portion 18 of the elongated rod 14 and a second coupling 90 connected to and extending from the second portion 22 of the elongated rod 14. The first coupling 86 and the second coupling 90 are oriented substantially perpendicular with respect to the elongated rod 14. The position device 10 also includes a first arm 94 and a second arm 98 removably coupled to the first coupling 86 and the second coupling 90, respectively. The first arm 94 and the second arm 98 substantially include the same structure, therefore only the first arm 94 is described herein. In another construction, the first arm 94 can be directly connected to and extend from the first portion 18 of the elongated rod 14 and, the second arm 98 can be directly connected to and extend from the second portion 22 of the elongated rod 14.

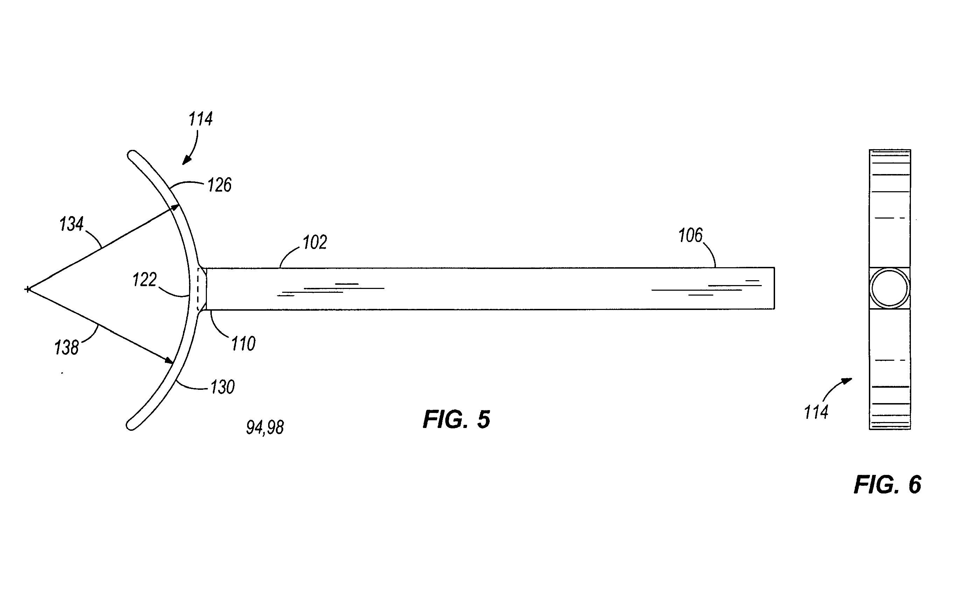

FIGS. 4 and 5 further illustrate the first arm 94 and the second arm 98. The first arm 94 includes an elongated rod 102 having a first end 106 and a second end 110. The elongated rod 102 includes a length generally defined by the distance between a person's head and shoulder. The first end 106 is adapted to be received within the first coupling 86 and be securely retained within the coupling with a friction fit. The elongated rod 102 has a substantially circular cross-section, but other shapes for the cross-section are also possible.

The first arm 94 also includes a base 114 coupled to the second end 110 of the rod 102. The base 114 is generally C-shaped and defines a recess 118 adapted to receive the shoulder of the patient. The base 114 includes a central portion 122 and a first extension 126 extending from the central portion 122 and a second extension 130 extending from the central portion 122. The first extension 126 defines a radius of curvature of a first length 134. The second extension 130 defines a radius of curvature of a second length 138. In one construction, the first length 134 and the second length 138 are substantially the same. In another construction, the first length 134 and the second length 138 are different. For example, the first length 134 can be about 4.25 inches and the second length 138 can be about 4.0 inches.

As illustrated in FIG. 2, the first arm 94 and the second arm 98 are spaced from one another based on a distance between the patient's left shoulder and right shoulder. As discussed above, the elongated rod 14 is adjusted to accommodate the width of the patient's shoulders. The area between the first arm 94 and the second arm 98 define a recess 142 adapted to receive the patient's head.

In operation, the device 10 is adjusted to accommodate the width of the patient's shoulders and is then applied to the patient's shoulders. When the patient is in a substantially horizontal position, the elongated rod 14 applies bilateral pressure to the patient's shoulders. In addition, the first arm and the second arm apply downward (i.e., toward the table the patient is lying on) pressure when pressure is applied to the elongated rod 14. When the downward pressure is applied to the elongated rod 14, the patient's shoulders are also pushed down and out of the way of the X-ray field. The device 10 allows for the acquisition of X-rays having C1 and C2, and C7 and C8, visible in a single X-ray.

## Claims

1. A shoulder press device (10) for applying pressure to a patient's shoulders during X-ray imaging, the device (10) comprising:
an elongated rod (14) having a first end (26) and a second end (38);
a first handle (66) coupled to the first end (26) of the elongated rod (14);
a second handle (70) coupled to the second end (38) of the elongated rod (14);
a first arm (94) including a first end (106) and a base (114), the first end (106) being coupled to the elongated rod (14), the first arm (94) oriented substantially perpendicular with respect to the elongated rod (14), the base (114) having a central portion (122), a first extension (126) extending from the central portion (122), and a second extension (130) extending from the central portion (122); and
a second arm (98) spaced from the first arm (94), the second arm (98) including a first end (106) and a base (114), the first end (106) being coupled to the elongated rod (14), the second arm (98) oriented substantially perpendicular with respect to the elongated rod (14), the base having a central portion (122), a first extension (126) extending from the central portion (122), and a second extension (130) extending from the central portion (122);
wherein, at the base (114) of each of the first and second arms (94, 98), the first extension (126) defines a radius of curvature of a first length (134), and the second extension (130) defines a radius of curvature of a second length (138); and
wherein the base (114) of each of the first and second arms (94, 98) defines a recess adapted to receive a shoulder of the patient;
the shoulder press device (10) **characterised in that**:
the elongated rod (14) is telescoping, having a first portion (18) and a second portion (22), the first portion (18) being telescopically adjustable within the second portion (22);
the first handle (66) includes an elongated portion (74), an enlarged portion (78) extending from the elongated portion (74), and an extension (82) extending from the enlarged portion (78), the extension (82) having a curvature following a downward path toward the second end (38) of the elongated rod (14);
the second handle (70) includes an elongated portion (74), an enlarged portion (78) extending from the elongated portion (74), and an extension (82) extending from the enlarged portion (78), the extension (82) having a curvature following a downward path toward the first end (26) of the elongated rod (14);
the first end (106) of the first arm (94) is coupled to the first portion (18) of the elongated rod (14);
the first end (106) of the second arm (98) is coupled to the second portion (22) of the elongated rod (14); and
at least a portion of the device (10) is comprised of radiolucent carbon fibre material.

2. A shoulder press device (10) as claimed in Claim 1, wherein:
the first end (106) of the first arm (94) is removably coupled to the first portion (18) of the elongated rod (14); and
the first end (106) of the second arm (98) is removably coupled to the second portion (22) of the elongated rod (14).

3. A shoulder press device (10) as claimed in Claim 1, wherein:
the first end (106) of the first arm (94) is directly connected to the first portion (18) of the elongated rod (14); and
the first end (106) of the second arm (98) is directly connected to the second portion (22) of the elongated rod (14).

4. A shoulder press device (10) as claimed in Claim 1, wherein, at the base (114) of each of the first and second arms (94, 98), the first length (134) is different from the second length (138).

5. A shoulder press device (10) as claimed in Claim 1, wherein, at the base (114) of each of the first and second arms (94, 98), the first length (134) and the second length (138) are substantially the same.

## Patentansprüche

1. Schulteranpressvorrichtung (10) zum Aufbringen von Druck auf die Schultern eines Patienten während einer Röntgenaufnahme, welche Vorrichtung (10) folgendes aufweist:
einen länglichen Stab (14) mit einem ersten Ende (26) und einem zweiten Ende (38);
einen ersten Griff (66), der mit dem ersten Ende (26) des länglichen Stabs (14) verbunden ist;
einen zweiten Griff (70), der mit dem zweiten Ende (38) des länglichen Stabs (14) verbunden ist;
einen ersten Arm (94) mit einem ersten Ende (106) und einer Basis (114), wobei das erste Ende (106) mit dem länglichen Stab (14) verbunden ist, der erste Arm (94) im Wesentlichen senkrecht zum länglichen Stab (14) ausgerichtet ist, die Basis (114) einen Mittelteil (122) aufweist, sich eine erste Verlängerung (126) vom Mittelteil (122) weg erstreckt und sich eine zweite Verlängerung (130) vom Mittelteil (122) weg erstreckt; und
einen vom ersten Arm (94) beabstandeten zweiten Arm (98), welcher zweite Arm (98) ein erstes Ende (106) und eine Basis (114) aufweist, wobei das erste Ende (106) mit dem länglichen Stab (14) verbunden ist, der zweite Arm (98) im Wesentlichen senkrecht zum länglichen Stab (14) ausgerichtet ist, die Basis einen Mittelteil (122) aufweist, sich eine erste Verlängerung (126) vom Mittelteil (122) weg erstreckt und sich eine zweite Verlängerung (130) vom Mittelteil (122) weg erstreckt;
wobei die erste Verlängerung (126) an der Basis (114) jedes des ersten und zweiten Arms (94, 98) einen Krümmungsradius mit einer ersten Länge (134) und die zweite Verlängerung (130) einen Krümmungsradius mit einer zweiten Länge (138) festlegt; und
wobei die Basis (114) jedes des ersten und zweiten Arms (94, 98) eine Ausnehmung festlegt, die zur Aufnahme einer Schulter des Patienten eingerichtet ist;
wobei die Schulteranpressvorrichtung (10) **dadurch gekennzeichnet ist, dass**:
der längliche Stab (14) teleskopierbar ist und einen ersten Teil (18) und einen zweiten Teil (22) aufweist, wobei der erste Teil (18) innerhalb des zweiten Teils (22) teleskopisch verschiebbar ist;
der erste Griff (66) einen länglichen Teil (74), einen sich vom länglichen Teil (74) weg erstreckenden, verbreiterten Teil (78) und eine sich vom verbreiterten Teil (78) weg erstreckende Verlängerung (82) aufweist, wobei die Verlängerung (82) eine Krümmung aufweist, die einem abwärts gerichteten Pfad zum zweiten Ende (38) des länglichen Stabs (14) folgt;
der zweite Griff (70) einen länglichen Teil (74), einen sich vom länglichen Teil (74) weg erstreckenden, verbreiterten Teil (78) und eine sich vom verbreiterten Teil (78) weg erstreckende Verlängerung (82) aufweist, wobei die Verlängerung (82) eine Krümmung aufweist, die einem abwärts gerichteten Pfad zum ersten Ende (26) des länglichen Stabs (14) folgt;
das erste Ende (106) des ersten Arms (94) mit dem ersten Teil (18) des länglichen Stabs (14) verbunden ist;
das erste Ende (106) des zweiten Arms (98) mit dem zweiten Teil (22) des länglichen Stabs (14) verbunden ist; und
zumindest ein Teil der Vorrichtung (10) aus einem strahlendurchlässigen Kohlefasermaterial besteht.

2. Schulteranpressvorrichtung (10) nach Anspruch 1, wobei:
das erste Ende (106) des ersten Arms (94) lösbar mit dem ersten Teil (18) des länglichen Stabs (14) verbunden ist; und
das erste Ende (106) des zweiten Arms (98) lösbar mit dem zweiten Teil (22) des länglichen Stabs (14) verbunden ist.

3. Schulteranpressvorrichtung (10) nach Anspruch 1, wobei:
das erste Ende (106) des ersten Arms (94) direkt mit dem ersten Teil (18) des länglichen Stabs (14) verbunden ist; und
das erste Ende (106) des zweiten Arms (98) direkt mit dem zweiten Teil (22) des länglichen Stabs (14) verbunden ist.

4. Schulteranpressvorrichtung (10) nach Anspruch 1, wobei an der Basis (114) jedes des ersten und zweiten Arms (94, 98) die erste Länge (134) von der zweiten Länge (138) unterschiedlich ist.

5. Schulteranpressvorrichtung (10) nach Anspruch 1, wobei an der Basis (114) jedes des ersten und zweiten Arms (94, 98) die erste Länge (134) und die zweite Länge (138) im Wesentlichen gleich sind.

## Revendications

1. Dispositif de presse pour épaule (10) visant à appliquer une pression aux épaules d'un patient pendant une imagerie aux rayons X, le dispositif (10) comprenant :
une tige allongée (14) ayant une première extrémité (26) et une seconde extrémité (38) ;
une première poignée (66) raccordée à la première extrémité (26) de la tige allongée (14) ;
une seconde poignée (70) raccordée à la seconde extrémité (38) de la tige allongée (14) ;
un premier bras (94) comprenant une première extrémité (106) et une base (114), la première extrémité (106) étant raccordée à la tige allongée (14), le premier bras (94) étant orienté sensiblement perpendiculairement à la tige allongée (14), la base (114) ayant une portion centrale (122), une première extension (126) s'étendant depuis la portion centrale (122) et une seconde extension (130) s'étendant depuis la portion centrale (122) ; et
un second bras (98) espacé depuis le premier bras (94), le second bras (98) comprenant une première extrémité (106) et une base (114), la première extrémité (106) étant raccordée à la tige allongée (14), le second bras (98) orienté sensiblement perpendiculairement relativement à la tige allongée (14), la base ayant une portion centrale (122), une première extension (126) s'étendant depuis la portion centrale (122) et une seconde extension (130) s'étendant depuis la portion centrale (122) ;
dans lequel, à la base (114) de chacun des premier et second bras (94, 98), la première extension (126) définit un rayon de courbure d'une première longueur (134) et la seconde extension (130) définit un rayon de courbure d'une seconde longueur (138) ; et
dans lequel, la base (114) de chacun des premier et second bras (94, 98) définit une cavité adaptée pour recevoir une épaule du patient ;
le dispositif de presse d'épaule (10), **caractérisé en ce que** :
la tige allongée (14) est télescopique, et dotée d'une première portion (18) et d'une seconde portion (22), la première portion (18) étant télescopiquement ajustable dans la seconde portion (22) ;
la première poignée (66) comprend une portion allongée (74), une portion élargie (78) s'étendant depuis la portion allongée (74), et une extension (82) s'étendant depuis la portion élargie (78), l'extension (82) ayant une courbure qui suit un chemin vers le bas, vers la seconde extrémité (38) de la tige allongée (14) ;
la seconde poignée (70) comprend une portion allongée (74), une portion élargie (78) s'étendant depuis la portion allongée (74), et une extension (82) s'étendant depuis la portion élargie (78), l'extension (82) ayant une courbure qui suit un chemin vers le bas vers la première extrémité (26) de la tige allongée (14) ;
la première extrémité (106) du premier bras (94) est raccordée à la première portion (18) de la tige allongée (14) ;
la première extrémité (106) du second bras (98) est raccordée à la seconde portion (22) de la tige allongée (14) ; et
au moins une portion du dispositif (10) comprend un matériau en fibre de carbone perméable aux rayons X.

2. Dispositif de presse pour épaule (10) selon la revendication 1, dans lequel :
la première extrémité (106) du premier bras (94) est raccordée de manière amovible à la première portion (18) de la tige allongée (14) ; et
la première extrémité (106) du second bras (98) est raccordée de manière amovible à la seconde portion (22) de la tige allongée (14).

3. Dispositif de presse pour épaule (10) selon la revendication 1, dans lequel :
la première extrémité (106) du premier bras (94) est directement raccordée à la première portion (18) de la tige allongée (14) ; et
la première extrémité (106) du second bras (98) est directement raccordée à la seconde portion (22) de la tige allongée (14).

4. Dispositif de presse pour épaule (10) selon la revendication 1, dans lequel, à la base (114) de chacun des premier et second bras (94, 98), la première longueur (134) est différente de la seconde longueur (138).

5. Dispositif de presse pour épaule (10) selon la revendication 1, dans lequel, à la base (114) de chacun des premier et second bras (94, 98), la première longueur (134) et la seconde longueur (138) sont sensiblement les mêmes.
